# EUROPÄISCHE PATENTSCHRIFT

(11) **EP 2 604 294 B1**
(45) Veröffentlichungstag und Bekanntmachung des Hinweises auf die Patenterteilung: **09.12.2015**
(21) Anmeldenummer: 12197245.9
(22) Anmeldetag: 14.12.2012
(51) Int. Cl.: A61L 2/20, B65B 55/10, B01B 1/00

(54) **Verdampfer mir Drainageleitung**
Evaporator with drain
Evaporateur avec drainage

(30) Priorität: 14.12.2011 DE 102011056438
(43) Veröffentlichungstag der Anmeldung: 19.06.2013
(73) Patentinhaber: Krones AG, 93073 Neutraubling (DE)
(72) Erfinder: Söllner, Jürgen, 93073 Neutraubling (DE); Bauer, Sascha, 93073 Neutraubling (DE); Vogelsang, Christian, 93073 Neutraubling (DE)
(74) Vertreter: Bittner, Bernhard

(56) Entgegenhaltungen:
- EP-A1- 2 286 846
- WO-A1-2009/132686
- WO-A1-2011/038487
- WO-A2-2008/063252
- WO-A2-2009/013226
- US-A- 3 326 202

## Beschreibung

Die Erfindung betrifft eine Verdampfervorrichtung zum Sterilisieren von Behältern umfassend eine Verdampferkammer mit einem Gehäuse, mit einer Zuführeinrichtung zum Zuführen eines gasförmigen Mediums in die Verdampferkammer, mit einer Einspritzeinrichtung zum Einspritzen einer flüssigen Wasserstoffperoxidlösung in die Verdampferkammer, mit einer Heizeinrichtung zum Verdampfen der flüssigen Wasserstoffperoxidlösung innerhalb der Verdampferkammer, mit einer Abführeinrichtung zum Abführen eines sich aus dem gasförmigen Medium und der flüssigen Wasserstoffperoxidlösung zusammensetzendes Sterilisationsgemisches und mit einer Drainageeinrichtung zum Ableiten zumindest von nicht verdampfter, flüssiger Wasserstoffperoxidlösung aus der Verdampferkammer, bei welcher die Abführeinrichtung eine von der Verdampferkammer wegführende Abführleitung aufweist, um das Sterilisationsgemisch an einer Sterilisationseinrichtung bereitzustellen, und bei welcher die Drainageeinrichtung eine von der Verdampferkammer wegführende Drainageleitung aufweist, um die nicht verdampfte, flüssige Wasserstoffperoxidlösung zur weiteren Verwendung bereitzustellen.

Auch betrifft die Erfindung ein Verfahren zum Betreiben einer Verdampfervorrichtung zum Sterilisieren von Behältern, bei welchem einer Verdampferkammer sowohl ein Volumengasstrom eines gasförmigen Mediums als auch eine flüssige Wasserstoffperoxidlösung zugeführt wird, bei welchem die flüssige Wasserstoffperoxidlösung innerhalb der Verdampferkammer zu einem Sterilisationsgemisch verdampft wird, bei welchem das Sterilisationsgemisch von dem Volumengasstrom mittels einer Abführeinrichtung aus der Verdampferkammer abgeführt und über eine Abführleitung der Abführeinrichtung an einer Sterilisationseinrichtung zur weiteren Verwendung bereitgestellt wird, und bei welchem nicht verdampfte, flüssige Wasserstoffperoxidlösung aus der Verdampferkammer mittels einer Drainageeinrichtung abgeleitet wird, um die Gefahr einer Detonation der nicht verdampften, flüssigen Wasserstoffperoxidlösung innerhalb der Verdampfervorrichtung zu verhindern.

Wasserstoffperoxid-Verdampfervorrichtungen, kurz H₂0₂-Verdampfer, sind aus dem Stand der Technik vielfältig bekannt und werden auch zu Sterilisationszwecken insbesondere von Behältern, wie von aus Kunststoffvorformlingen expandierten Kunststoffbehältern, diesbezüglichen Verschlüssen, aber auch von kompletten Reinräumen an Getränkebehälterherstellanlagen und/oder Getränkebehälterabfüllanlagen vorteilhaft eingesetzt.

Bei den Behältern kann es sich sowohl um Kunststoffbehälter wie Kunststoffflaschen oder Kunststoffvorformlinge handeln als auch um Behälter aus anderen Materialien wie Glas oder Verbundstoffen.

Hierzu wird einer Verdampfervorrichtung zugeführtes flüssiges Wasserstoffperoxid (H₂0₂) innerhalb einer Verdampferkammer der Verdampfervorrichtung verdampft. Die verdampfte Wasserstoffperoxidlösung vermischt sich mit einem ebenfalls in die Verdampferkammer eingeleiteten gasförmigen Medium, wie etwa Umgebungsluft, und es entsteht hierbei ein H₂0₂-Luft-Sterilisationsgemisch, welches einer Abführeinrichtung an der Verdampfervorrichtung zur weiteren Verwendung bereitgestellt wird. Zur Erzeugung eines derartigen H₂0₂-Luft-Sterilisationsgemisches wird eine 35% handelsübliche flüssige Wasserstoffperoxidlösung auf eine auf etwa 180 °C aufgeheizte Verdampferoberfläche einer Heizeinrichtung gesprüht. Beim Kontakt der flüssigen Wasserstoffperoxidlösung mit der heißen Verdampferoberfläche wird der flüssigen Wasserstoffperoxidlösung die erforderliche Energie zugeführt, damit sie innerhalb sehr kurzer Zeit in die Dampfphase übergehen kann. Mit Hilfe eines eingespeisten Luftvolumenstroms kann es dann aus der Verdampferkammer abgeführt werden. Die flüssige Wasserstoffperoxidlösung besteht im Wesentlichen aus zwei Phasen, nämlich dem reinen Wasserstoffperoxid und Wasser. Der Siedepunkt des flüssigen Wasserstoffperoxids ist hierbei stark vom Anteil des reinen Wasserstoffperoxids abhängig und er liegt bei der eingangs genannten handelsüblichen 35%igen Wasserstoffperoxidlösung bei etwa 108 °C. Mit zunehmender Konzentration an reinem Wasserstoffperoxid erhöht sich der Siedepunkt des flüssigen Wasserstoffperoxids allgemein. Der Siedepunkt liegt bei reinem Wasserstoffperoxid etwa bei 150 °C; also immer noch deutlich unter der normalen Betriebsoberflächentemperatur der Heizeinrichtung.

Solange die aufgeheizte Verdampferoberfläche der Heizeinrichtung die erforderliche Oberflächentemperatur aufweist, bevor die flüssige Wasserstoffperoxidlösung darauf dosiert wird, wird die flüssige Wasserstoffperoxidlösung immer vollständig verdampft und sie kann als Sterilisationsgemisch ordnungsgemäß mittels der Abführeinrichtung aus der Verdampferkammer abgeführt werden.

In einem gattungsgemäßen Wasserstoffperoxid-Verdampfer kann es unter bestimmten Umständen jedoch zu einer gefährlichen Aufkonzentrierung des Wasserstoffperoxids kommen. Kommt es beispielsweise dazu, dass auf eine kalte oder nur unzureichend aufgeheizte Verdampferoberfläche der Heizeinrichtung flüssige Wasserstoffperoxidlösung dosiert wird, besteht die Gefahr, dass sich die flüssige Wasserstoffperoxidlösung in einem kritischen Maße in der Verdampferkammer ansammelt.

Wird nun die Heizeinrichtung aktiviert oder die Verdampferoberflächentemperatur in Richtung 180 °C erhöht, wird die flüssige Wasserstoffperoxidlösung nur verzögert über den Siedepunkt von Wasser erhitzt, so dass die Lösung mit Wasserstoffperoxid aufkonzentriert wird. Da dieser Heizvorgang aufgrund der relativ hohen installierten Heizleitung sehr rasch vonstatten geht, zum vollständigem Verdampfen von 1 kg flüssiger Wasserstoffperoxidlösung hinsichtlich eines 5 kg Wasserstoffperoxid-Krones-Standardverdampfers sind etwa 4,5 Minuten erforderlich, kommt es in der Wasserstoffperoxidlösung kaum zu Zersetzungen. Dies führt in der Regel dazu, dass sich das Wasserstoffperoxid in der Lösung auf eine Konzentration von bis über 90% aufkonzentrieren kann.

Eine derart aufkonzentrierte Wasserstoffperoxidlösung mit einem über 90% Wasserstoffperoxid-Anteil kann jedoch unter Einwirkung von Energie, beispielsweise aus der Verdampferoberfläche, schnell explodieren/detonieren. Da ein hieraus resultierender schlagartiger Druckaufbau mit den vorhandenen Sicherheitseinrichtungen meist nicht mehr vollständig beherrscht werden kann, besteht die Gefahr, dass die gesamte Verdampfervorrichtung zerberstet und damit zerstört wird.

Da hierbei auch ein enormes Gefahrenpotential für Menschen besteht, die sich in unmittelbarer Nähe zur Verdampfervorrichtung aufhalten, muss eine kritische Ansammlung einer aufkonzentrierten Wasserstoffperoxidlösung unbedingt vermieden werden. In der noch unveröffentlichten DE 10 2010 027 076.8 wird ein Verdampfer zum Sterilisieren von Kunststoffbehältnissen beschrieben. Der Gegenstand dieser Offenbarung wird durch Bezugnahme vollständig auch zum Gegenstand der vorliegenden Anmeldung gemacht.

Aus der US 3 326 202 A ist eine Warmwasserbereitungs- und Reinigungsvorrichtung bekannt. Das zu reinigende oder zu erwärmende Wasser wird über Düsen in eine Zwischenkammer gesprüht und dort und insbesondere auf einer Heizfläche mit Hilfe eines heißen Gases in Dampf umgewandelt. Der so erzeugte Dampf strömt über Durchgänge in eine Außenkammer und wird über eine Auslassöffnung in einen Auslasskanal eingeleitet. Hier wird der Dampf durch kaltes Wasser, welches in einem Abschnitt dem Gas entgegen fließt, kondensiert. Fremdstoffe und das Kondensat sinken nun durch die Gravitation auf die Bodenfläche. Eine solche Warmwasserbereitungs- und Reinigungsvorrichtung ist nicht für die Erzeugung und Behandlung von Peroxiden geeignet, da sie bei einer solchen zweckfremden Verwendung insbesondere in der Zwischenkammer die Anreicherung von nicht in die Gasphase übergegangenen Peroxiden ermöglicht, was zu einer Explosion führen könnte.

Zum Vermeiden einer kritischen Ansammlung einer derartigen aufkonzentrierten Wasserstoffperoxidlösung innerhalb der Verdampferkammer ist es im Stand der Technik bereits üblich, die aufkonzentrierte Wasserstoffperoxidlösung mittels einer geeigneten Sammeleinrichtung zu sammeln und gegebenenfalls aufbereitet der Verdampferkammer später erneut zuzuführen. Derartige Ableit- und Sammelbehälter bauen oftmals recht aufwendig, was letztendlich auch den Aufwand von Wartungsarbeiten an der Verdampfervorrichtung insgesamt erhöht. Hierdurch verteuert sich nicht nur die Herstellung solcher Verdampfervorrichtungen sondern auch deren Unterhalt.

Es ist Aufgabe der Erfindung, die vorstehend genannten Nachteile durch geeignete konstruktive Maßnahmen zu beseitigen, wodurch die Verdampfervorrichtung auch bestens geeignet ist, länderspezifische Vorschriften hinsichtlich Anlagen- und Produktsicherheit, wie beispielsweise die Richtlinien der FDA, zu erfüllen.

Die Aufgabe der Erfindung wird einerseits von einer Verdampfervorrichtung zum Sterilisieren von Behältern umfassend eine Verdampferkammer mit einem Gehäuse, mit einer Zuführeinrichtung zum Zuführen eines gasförmigen Mediums in die Verdampferkammer, mit einer Einspritzeinrichtung zum Einspritzen einer Wasserstoffperoxidlösung in die Verdampferkammer, mit einer Heizeinrichtung zum Verdampfen der Wasserstoffperoxidlösung innerhalb der Verdampferkammer, mit einer Abführeinrichtung zum Abführen eines sich aus dem gasförmigen Medium und der Wasserstoffperoxidlösung zusammensetzendes Sterilisationsgemisches und mit einer Drainageeinrichtung zum Ableiten zumindest von nicht verdampfter Wasserstoffperoxidlösung aus der Verdampferkammer gelöst, bei welcher die Abführeinrichtung eine von der Verdampferkammer wegführenden Abführleitung aufweist, um das Sterilisationsgemisch an einer Sterilisationseinrichtung bereitzustellen, und bei welcher die Drainageeinrichtung eine von der Verdampferkammer wegführende Drainageleitung aufweist, um nicht verdampfte Wasserstoffperoxidlösung zur weiteren Verwendung bereitzustellen, wobei die Drainageleitung der Drainageeinrichtung mit ihrer Eingangsöffnung an der Verdampferoberfläche und somit unterseitig der Verdampferkammer angeordnet ist und - bevorzugt direkt - in die Abführleitung zum Abführen des Sterilisationsgemisches mündet, um zumindest die in der Verdampferkammer nicht verdampfte Wasserstoffperoxidlösung aus der Verdampferkammer direkt in die Abführleitung abzuführen.

Mit dem Begriff "gasförmiges Medium" ist vorliegend vorzugsweise Luft gemeint, welche als Volumengasstrom der Verdampferkammer zugeführt wird, um hierüber das Sterilisationsgemisch aus der Verdampferkammer heraus zu transportieren. Es kann jedoch auch jedes andere gasförmige Medium verwendet werden, wenn dies als vorteilhaft erachtet wird.

Der Begriff "flüssige Wasserstoffperoxidlösung" beschreibt im Sinne der Erfindung eine im Wesentlichen zweiphasige Lösung mit einer ersten Phase umfassend Wasser und einer zweiten Phase umfassend Wasserstoffperoxid. Für die vorliegenden Sterilisationszwecke reicht ein 35%-Anteil an Wasserstoffperoxid hinsichtlich der hier verwendeten handelsüblichen flüssigen Wasserstoffperoxidlösung, was jedoch andere Mengenverhältnisse nicht ausschließt. Daneben wäre es auch möglich, andere verdampfbare Sterilisationsmedien wie etwa Peressigsäure einzusetzen, so dass die Erfindung allgemein auf eine (insbesondere durch Wärmeeinwirkung verdampfbare) Sterilisationsmittellösung anwendbar ist.

Mit der Bezeichnung "Sterilisationsgemisch" ist ein Gemisch aus verdampfter Wasserstoffperoxidlösung (oder allgemein Sterilisationsmittellösung) und dem Volumengasstrom aus dem gasförmigen Medium beschrieben, wobei das Sterilisationsgemisch als Sterilisationsgemischvolumenstrom mit der Abführeinrichtung bzw. dessen Abführleitung aus der Verdampferkammer heraus transportiert wird.

Erfindungsgemäß mündet die Drainageleitung in die Abführleitung zum Abführen des Sterilisationsgemisches, welche bisher lediglich dafür vorgesehen war, das mittels der Verdampfervorrichtung erzeugte Sterilisationsgemisch aus der Verdampferkammer herauszuführen und an einer Sterilisationseinrichtung zum Sterilisieren etwa von Kunststoffbehältern bereitzustellen, wodurch die Gefahr einer unbeabsichtigten Detonation von aufkonzentrierter Wasserstoffperoxidlösung wesentlich verringert werden kann, da an der Abführeinrichtung ausreichend hohe Temperaturen eben nicht vorherrschen, welche eine Zündung des aufkonzentrierten Wasserstoffperoxids verursachen könnten.

Insofern wird die Aufgabe der Erfindung andererseits auch von einem Verfahren zum Betreiben einer Verdampfervorrichtung zum Sterilisieren von Behältern gelöst, bei welchem einer Verdampferkammer sowohl ein Volumengasstrom eines gasförmigen Mediums als auch eine flüssige Wasserstoffperoxidlösung zugeführt wird, bei welchem die flüssige Wasserstoffperoxidlösung innerhalb der Verdampferkammer zu einem Sterilisationsgemisch verdampft wird, bei welchem das Sterilisationsgemisch von dem Volumengasstrom mittels einer Abführeinrichtung aus der Verdampferkammer abgeführt und über eine Abführleitung der Abführeinrichtung an einer Sterilisationseinrichtung zur weiteren Verwendung bereitgestellt wird, und bei welchem nicht verdampfte, flüssige Wasserstoffperoxidlösung aus der Verdampferkammer mittels einer Drainageeinrichtung abgeleitet wird, um die Gefahr einer Detonation der nicht verdampften, flüssigen Wasserstoffperoxidlösung innerhalb der Verdampfervorrichtung zu verhindern, wobei sich das Verfahren dadurch auszeichnet, dass die nicht verdampfte, flüssige Wasserstoffperoxidlösung mittels der Drainageeinrichtung, bei der eine Drainageleitung mit ihrer Eingangsöffnung an der Verdampferoberfläche und somit unterseitig der Verdampferkammer angeordnet ist, direkt in die Abführleitung zum Abführen des Sterilisationsgemisches eingeleitet wird.

Hierdurch kann die Gefahr einer Detonation einer nicht verdampften, flüssigen Wasserstoffperoxidlösung drastisch reduziert werden, da eine solche Detonation innerhalb der Abführeinrichtung aufgrund zu niedriger Temperaturen ausgeschlossen werden kann. Vielmehr kann die in die Abführleitung eingeleitete nicht verdampfte, flüssige Wasserstoffperoxidlösung von dem die Abführleitung durchströmenden Sterilisationsgemischvolumenstrom zumindest noch teilweise aufgenommen werden.

Eine besonders einfach bauende Ausführungsvariante sieht vor, dass die Drainageeinrichtung zumindest abschnittsweise die Abführleitung zum Abführen des Sterilisationsgemisches umfasst. Hierdurch kann die Verdampfervorrichtung konstruktiv weiter sehr gut vereinfacht werden. Vorteilhafter Weise ist es somit besonders gut möglich, auf eine Vielzahl an sonst erforderlichen Leitungen zu verzichten, so dass sich insbesondere der Aufbau der Drainageeinrichtung und damit auch der Aufbau der vorliegenden Verdampfervorrichtung gegenüber gattungsgemäßen Verdampfervorrichtungen sehr weit vereinfachen lässt.

Es versteht sich, dass die Drainageleitung und die Abführleitung nahezu in beliebiger Weise zusammengeführt werden können. Mündet die Drainageleitung jedoch außerhalb der Verdampfervorrichtung in die Abführleitung zum Abführen des Sterilisationsgemisches, kann eine Detonation aufgrund einer kritischen Temperaturerhöhung ausgeschlossen werden, selbst wenn sich im Bereich einer Mündungsöffnung nicht verdampfte aufkonzentrierte Wasserstoffperoxidlösung ansammeln würde.

Vorteilhaft ist die Verdampferkammer bzw. die Heizeinrichtung an einem höheren Punkt der Vorrichtung angeordnet als die Drainageleitung und besonders bevorzugt ist die Verdampferkammer am höchsten Punkt der Vorrichtung angeordnet, so dass auf zusätzliche Einbauten zum Leiten der flüssigen Wasserstoffperoxidlösung (oder allgemein der Sterilisationsmittellösung) verzichtet werden kann.

Um die Sicherheit der Ableitung der nicht verdampften Wasserstoffperoxidlösung weiter erhöhen zu können, ist es vorteilhaft, wenn eine Mündungsöffnung der Drainageleitung an der Abführleitung zum Abführen des Sterilisationsgemisches oberhalb eines tiefsten Abführleitungsabschnittes angeordnet ist.

Des Weiteren ist es vorteilhaft, wenn ein Verdampferkammeranschluss der Drainageeinrichtung unterhalb der Heizeinrichtung an dem Gehäuse der Verdampferkammer angeschlossen ist. Hierdurch kann gewährleistet werden, dass nicht verdampfte Wasserstoffperoxidlösung außerordentlich betriebssicher aus der Verdampferkammer abgeleitet und in die Abführleitung zum Abführen des Sterilisationsgemisches des erzeugten Sterilisationsgemisches eingeleitet werden kann.

Ein Einleiten von nicht verdampfter Wasserstoffperoxidlösung in die Abführleitung zum Abführen des Sterilisationsgemisches des erzeugten Sterilisationsgemisches kann weiter verbessert werden, wenn ein Verdampferkammeranschluss der Drainageeinrichtung an einer für nicht verdampfte Wasserstoffperoxidlösung vorgesehenen Sammeleinrichtung mit einer Senke angeordnet ist. Vorzugsweise ist hierbei zumindest die Senke und idealerweise ist die Sammeleinrichtung ebenfalls unterhalb der Verdampferkammer angeordnet.

Zeichnet sich die Verdampfervorrichtung kumulativ durch eine Steuereinrichtung zum Steuern der Einspritzeinrichtung oder einer Dosiereinheit hiervon bezüglich einer Dosierung der Wasserstoffperoxidlösung in die Verdampferkammer aus, wobei die Steuereinrichtung ein Messelement einer Detektiereinheit zum Detektieren einer Menge an nicht verdampfter Wasserstoffperoxidlösung an der Drainageeinrichtung umfasst, kann die Betriebssicherheit an der Verdampfervorrichtung weiter erhöht werden, da ein Einspritzen von weiterer Wasserstoffperoxidlösung in die Verdampferkammer hinein vorteilhaft Weise unmittelbar gestoppt werden kann, sollte sich zu viel nicht verdampfte Wasserstoffperoxidlösung in der Drainageeinrichtung ansammeln, welche in einem ausreichend sicheren Maße nicht mehr in die Abführleitung zum Abführen des Sterilisationsgemisches des erzeugten Sterilisationsgemisches eingeleitet werden kann.

Es wird jedoch darauf hingewiesen, dass diese Ausführungsform auch unabhängig von der in die Abführleitung mündenden Drainageleitung anwendbar ist.

Die Anmelderin behält sich daher vor, Schutz zu beanspruchen für eine Vorrichtung nach dem Oberbegriff von Anspruch 1, bei der in der Drainageleitung eine Detektoreinrichtung zum Detektieren einer Menge an Wasserstoffperoxidlösung (allgemein an Sterilisationsmittellösung) angeordnet ist. Vorteilhaft ist, wie oben erwähnt, auch eine Steuereinrichtung vorgesehen, welche die Zufuhr der Wasserstoffperoxidlösung in den Verdampfer steuert, wobei die Steuereinrichtung vorteilhaft die Zufuhr auch in Abhängigkeit von einer von der Detektionseinrichtung erfassten Menge an Wasserstoffperoxid steuert. Zusätzlich oder daneben wäre es möglich, dass etwa auch die Heizeinrichtung in Abhängigkeit von einer von der Detektoreinrichtung erfassten Menge an Wasserstoffperoxid gesteuert wird.

Eine diesbezüglich besonders vorteilhafte Ausführungsvariante sieht vor, dass das Messelement in einem Abschnitt der Drainageleitung zwischen der Verdampferkammer und einer Mündungsöffnung zwischen der Drainageleitung und der Abführleitung angeordnet ist. Hierdurch kann die Gefahr eines Nichterfassens von nicht verdampfter Wasserstoffperoxidlösung außerordentlich gut verringert werden.

Weitere Vorteile, Ziele und Eigenschaften vorliegender Erfindung werden anhand anliegender Zeichnung und nachfolgender Beschreibung erläutert, in welchen beispielhaft zwei Verdampfervorrichtungen mit jeweils einer in eine Abführleitung zum Abführen eines Sterilisationsgemisches mündenden Drainageleitung zum Ableiten von nicht verdampfter Wasserstoffperoxidlösung dargestellt und beschrieben sind. In der Zeichnung zeigen:

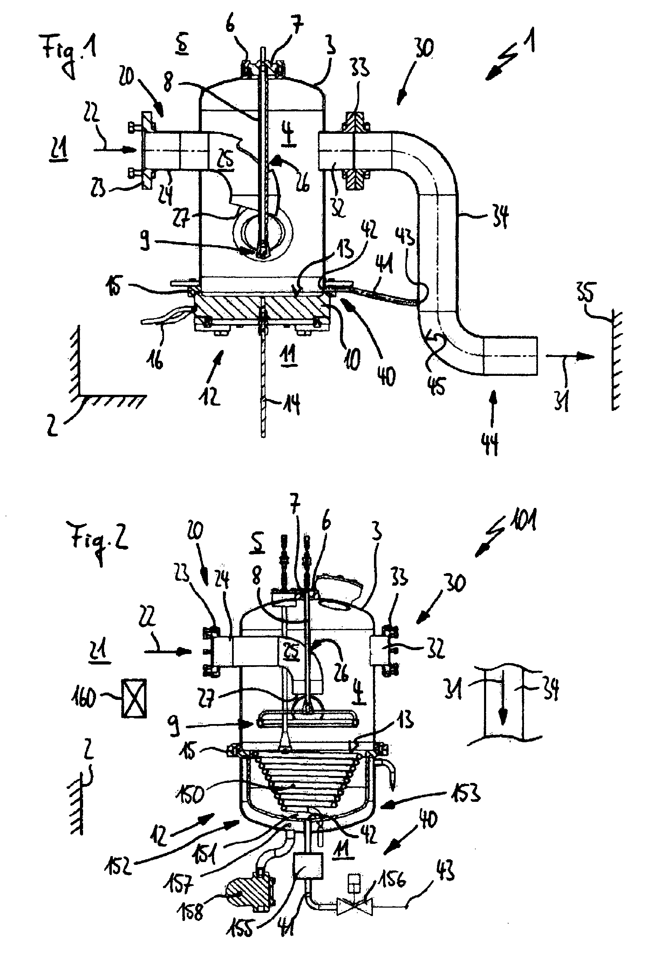

- Figur 1: schematisch eine Ansicht einer Verdampfervorrichtung, bei welcher eine Drainageleitung zum Ableiten zumindest von nicht verdampfter, flüssiger Wasserstoffperoxidlösung direkt in eine Abführleitung eines Sterilisationsgemisches mündet, um zumindest die in einer Verdampferkammer nicht verdampfte, flüssige Wasserstoffperoxidlösung aus der Verdampferkammer direkt in die Abführleitung zum Abführen des Sterilisationsgemisches abzuführen; und
- Figur 2: schematisch eine Ansicht einer weiteren Verdampfervorrichtung, bei welcher ebenfalls eine Drainageleitung zum Ableiten zumindest von nicht verdampfter, flüssiger Wasserstoffperoxidlösung in eine Abführleitung zum Abführen des Sterilisationsgemisches eines Sterilisationsgemisches mündet, wobei in der Drainageleitung noch ein Messelement zum Detektieren einer Menge an nicht verdampfter, flüssiger Wasserstoffperoxidlösung angeordnet ist.

Die in der Figur 1 gezeigte Verdampfervorrichtung 1 ist Bestandteil einer hier nicht weiter dargestellten Getränkebehälterherstellanlage 2, mittels welcher Kunststoffvorformlinge zu Kunststoffbehältern umgeformt werden können. Die Verdampfervorrichtung 1 umfasst eine mit einem Gehäuse 3 eingehauste Verdampferkammer 4. Oberseitig 5 des Gehäuses 3 ist mit Schrauben 6 ein Deckel 7 an das Gehäuse 3 angeschraubt. An dem Deckel 7 ist eine Einspritzeinrichtung 8 zum Einspritzen einer flüssigen Wasserstoffperoxidlösung in die Verdampferkammer 4 gehaltert. Die Einspritzeinrichtung 8 hängt hierbei senkrecht von oben in der Verdampferkammer 4 hinein. Sie ist mit ihren Einspritzdüsen 9 direkt oberhalb eines Heizplattenelements 10 einer unterseitig 11 der Verdampferkammer 4 liegenden Heizeinrichtung 12 zum Verdampfen der flüssigen Wasserstoffperoxidlösung platziert, so dass die flüssige Wasserstoffperoxidlösung von oben weitflächig auf eine Verdampferoberfläche 13 aufdosiert werden kann.

Um die Oberflächentemperatur an der Verdampferoberfläche 13 optimal überwachen und einstellen zu können, ist die Heizeinrichtung 12 mit einer Temperatursensoreinrichtung 14 ausgerüstet. Oberhalb an der Heizeinrichtung 12 ist ein Trägerring 15 vorgesehen, auf welchem wiederum das Gehäuse 3 abgestützt ist. Bevorzugt ist der Trägerring 15 aus einem Material hergestellt, welches sich durch einen möglichst geringen Wärmeleitquotienten auszeichnet, damit die von der Heizeinrichtung 12 abgegebene Wärmeenergie möglichst nicht oder nur vernachlässigbar gering auf das Gehäuse 3 übergeht. Das Bezugszeichen 16 kennzeichnet elektrische Zuführleitungen zum Versorgen der Heizeinrichtung 12. Die Heizeinrichtung könnte jedoch auch in anderer Weise, etwa mittels durchgeleitetem Dampf betrieben werden. Daneben könnte auch eine Kühlmittelversorgung zur genaueren Temperaturregelung vorgesehen sein.

Darüber hinaus ist an dem Gehäuse 3 eine Zuführeinrichtung 20 zum Zuführen eines gasförmigen Mediums, hier Umgebungsluft 21, vorgesehen, über welche der Verdampferkammer 4 ein Volumenluftstrom 22 zugeführt werden kann. Die Zuführeinrichtung 20 weist einen Flansch 23 auf, an welchem ein Zuluftsystem (hier nicht gezeigt) an einem ersten Kanalabschnitt 24 der Zuführeinrichtung 20 anschließbar ist. Im Inneren der Verdampferkammer 4 schließt sich an den ersten Kanalabschnitt 24 ein nach unten gekrümmter Kanalabschnitt 25 an, der oberseitig eine Seitenkanalöffnung 26 aufweist, durch welche hindurch die Einspritzeinrichtung 8 bis durch eine der Heizeinrichtung 12 zugewandten Kanalaustrittsöffnung 27 der Zuführeinrichtung 20 hindurch geführt ist. Hierdurch erfolgt die Strömungsführung des Volumenluftstroms 22 abschnittsweise in etwa parallel zur Einspritzeinrichtung 8.

Daneben kann durch die Seitenöffnung 26 auch ein Anteil des Volumenluftstroms 22 aus dem gekrümmten Kanalabschnitt 25 austreten und in Richtung einer Abführeinrichtung 30 zum Abführen eines Sterilisationsgemisches 31 gelangen.

Das Sterilisationsgemisch 31 setzt sich hierbei aus dem gasförmigen Medium und der mittels der Heizeinrichtung 12 innerhalb der Verdampferkammer 4 verdampften Wasserstoffperoxidlösung zusammen, wobei das Sterilisationsgemisch 31 als Sterilisationsgemischvolumenstrom über die Abführeinrichtung 30 aus der Verdampferkammer 4 heraus transportiert wird.

Die Abführeinrichtung 30 ist mit einem Anschlussstutzen 32 an dem Gehäuse 3 angeordnet, wobei der Anschlussstutzen 32 einen Anschlussflansch 33 aufweist, an welchem eine von der Verdampferkammer 4 wegführende Abführleitung 34 an die Verdampferkammer 4 angeflanscht ist. Mittels der Abführleitung 34 kann das Sterilisationsgemisch 31 problemlos an einer entfernten und hier nur schematisch dargestellten Sterilisationseinrichtung 35 der Getränkebehälterherstellanlage 2 zur weiteren Verwendung bereitgestellt werden.

Für den Fall, dass die eingespritzte flüssige Wasserstoffperoxidlösung an der Verdampferoberfläche 13 nicht vollständig verdampft wird, verfügt die Verdampfervorrichtung 1 noch über eine Drainageeinrichtung 40 zum Ableiten zumindest nicht verdampfter, flüssiger Wasserstoffperoxidlösung aus der Verdampferkammer 4 heraus. Hierbei ist eine Drainageleitung 41 der Drainageeinrichtung 40 mit ihrer Eingangsöffnung 42 an der Verdampferoberfläche 13 und somit unterseitig 11 der Verdampferkammer 4 angeordnet. Insofern kann besonders gut verhindert werden, dass sich an der Verdampferoberfläche 13 flüssige Wasserstoffperoxidlösung ansammeln kann, ohne dass die Heizeinrichtung 12 die zum vollständigen Verdampfen der flüssigen Wasserstoffperoxidlösung benötigte Temperatur an der Verdampferoberfläche 13 zur Verfügung gestellt hat. Hierdurch kann vermieden werden, dass sich das flüssige Wasserstoffperoxid zu einer leicht zündfähigen Lösung aufkonzentrieren kann.

Erfindungsgemäß mündet die Drainageleitung 41 direkt in die Abführleitung 34, so dass zumindest die in der Verdampferkammer 4 nicht verdampfte, flüssige Wasserstoffperoxidlösung aus der Verdampferkammer 4 direkt in die Abführleitung 34 abgeführt werden kann.

Hierdurch kann insbesondere eine nicht verdampfte, flüssige und gegebenenfalls schon aufkonzentrierte Wasserstoffperoxidlösung vorteilhaft von der heißen Verdampferoberfläche 13 direkt in die kühlere Abführleitung 34 abgeleitet und von dem Sterilisationsgemischvolumenstrom gekühlt und idealerweise zumindest teilweise von diesem aufgenommen und mitgeführt werden. Insofern kann sich eine weitere Aufbereitung und/oder Entsorgung der nicht verdampften, flüssigen und aufkonzentrierten Wasserstoffperoxidlösung zumindest teilweise erübrigen.

In diesem Ausführungsbeispiel mündet die Drainageleitung 41 mit ihrer Mündungsöffnung 43 außerhalb der Verdampferkammer 4 in die Abführleitung 34. Und zwar oberhalb eines tiefsten Abführleitungsabschnittes 44 der Abführleitung 34, so dass aus der Mündungsöffnung 43 in die Abführleitung 34 austretende nicht verdampfte, flüssige und aufkonzentrierte Wasserstoffperoxidlösung vorteilhafter Weise großflächig verteilt an einer Innenwandung 45 der Abführleitung 34 abfließen kann. Hierdurch kann die nicht verdampfte, flüssige und aufkonzentrierte Wasserstoffperoxidlösung besonders rasch unter eine kritische Temperatur abkühlen, so dass eine weitere Aufkonzentrierung der Lösung konstruktiv auf besonders einfache Weise verhindert werden kann.

Die Eingangsöffnung 42 der Drainageleitung liegt vorteilhaft höher als die Mündungsöffnung, sodass die Wasserstoffperoxidlösung unter Wirkung der Schwerkraft in die Abführleitung geführt werden kann.

Hiervon unbeeinträchtigt verbleiben vorzugsweise sämtliche ansonsten bekannte Primärmaßnahmen zur Vermeidung einer Kaltdosage der flüssigen Wasserstoffperoxidlösung bei einer nicht ausreichenden Verdampfungstemperatur an der Verdampferoberfläche 13, etwa durch Bloc-and-Bleed-Ventile an der Einspritzeinrichtung mit entsprechender Hardwareabschaltung.

Die in der Figur 2 gezeigte Verdampfervorrichtung 101 besitzt im Wesentlichen den gleichen Aufbau wie die Verdampfervorrichtung 1 aus der Figur 1. Insofern sind nachfolgend nur die wesentlichen Unterscheidungsmerkmale zwischen der Verdampfervorrichtung 101 und der Verdampfervorrichtung 1 beschrieben. Gleiche oder gleichwirkende Bauteile sind hierbei mit identischen Bezugszeichen versehen.

Die Verdampfervorrichtung 101 zeichnet sich insbesondere durch eine anders aufgebaute Drainageeinrichtung 40 aus, welche unterhalb (bzw. allgemein außerhalb) einer Verdampferkammer 4 angeordnet ist, um von einer Heizrohrleitungswendel 150 einer Heizeinrichtung 12 abgetropfte und nicht verdampfte, flüssige Wasserstoffperoxidlösung aus der Verdampferkammer 4 ableiten zu können. Die nicht verdampfte, flüssige Wasserstoffperoxidlösung wird unterseitig 11 der Verdampferkammer 4 in einer Senke 151 einer Sammeleinrichtung 152 gesammelt und sie gelangt von dort über eine Eingangsöffnung 42 in die Drainageleitung 41. Die Verdampfervorrichtung 101 ist unterseitig 11 mit einem doppelwandigen Klöpperboden 153 ausgestattet, der ebenfalls von der Heizeinrichtung 12 beheizbar ist, so dass dort von der Heizrohrleitungswendel 150 abgetropfte, nicht verdampfte Wasserstoffperoxidlösung zusätzlich noch verdampft werden kann. Durch die Heizrohrleitungswendel kann hier z.B. Dampf geführt werden, so dass es sich hier allgemein um eine Heizeinrichtung handelt, die von einem fließfähigen Medium durchflossen wird.

In der Drainageleitung 41 befindet sich eine Messsonde 155, welche die nicht verdampfte, flüssige Wasserstoffperoxidlösung innerhalb der Drainageleitung 41 detektiert.

Der Messsonde 155 nachgeschaltet ist noch ein Drainageventil 156. Durch dessen Öffnen können Betriebszustände der Verdampfervorrichtung 1 bei einer nicht aktiven Dosage von flüssiger Wasserstoffperoxidlösung zusätzlich zu den an sich bekannten Primärmaßnahmen abgesichert werden, um eine Kaltdosage besonders sicher zu vermeiden.

Die Drainageleitung 41 mündet, wie bei der Verdampfervorrichtung 1 aus der Figur 1, vorteilhaft, jedoch nicht notwendigerweise, ebenfalls direkt in eine Abführleitung 34 zum Abführen eines Sterilisationsgemisches 31. Somit kann das nicht verdampfte Wasserstoffperoxid besonders vorteilhaft aus der Verdampfervorrichtung 101 abgeleitet werden, so wie dies vorstehend bereits beschrieben ist.

Um auch ein sich an dem doppelwandigen Klöpperboden 153 zusätzlich gebildetes Kondensat vorteilhaft aus einem Zwischenraum 157 des doppelwandigen Klöpperboden 153 ableiten zu können, ist hierfür noch eine Ableiteinrichtung 158 vorgesehen.

Um die Einspritzeinrichtung 8 insbesondere in Abhängigkeit mittels von der Messsonde 155 gewonnen Daten steuern zu können, verfügt die Verdampfervorrichtung 101 noch über eine entsprechende Steuereinrichtung 160. Beispielsweise können die Steuereinrichtung 160 die Funktion der Einspritzeinrichtung 8 blockieren, so dass bei einer kritischen Menge an detektierter nicht verdampfter, flüssiger Wasserstoffperoxidlösung an der Messsonde 155 die Einspritzung von neuer flüssiger Wasserstoffperoxidlösung in die Verdampferkammer 4 unterbunden ist.

Die vorstehend beschriebenen Verdampfervorrichtungen 1 und 101 eignen sich besonders gut für den Einsatz in Getränkebehälterherstellanlagen 2 unter Reinraumbedingungen mit einer Trockensterilisation auf Basis von Wasserstoffperoxid.

### Bezugszeichenliste

- 1: Verdampfervorrichtung
- 2: Getränkebehälterherstellanlage
- 3: Gehäuse
- 4: Verdampferkammer
- 5: oberseitig
- 6: Schrauben
- 7: Deckel
- 8: Einspritzeinrichtung
- 9: Einspritzdüsen
- 10: Heizplattenelement
- 11: unterseitig
- 12: Heizeinrichtung
- 13: Verdampferoberfläche
- 14: Temperatursensoreinrichtung
- 15: Trägerring
- 16: elektrische Versorgungsleitung
- 20: Zuführeinrichtung
- 21: Umgebungsluft
- 22: Volumenluftstrom
- 23: Flansch
- 24: erster Kanalabschnitt
- 25: gekrümmter Kanalabschnitt
- 26: Seitenkanalöffnung
- 27: Kanalaustrittsöffnung
- 30: Abführeinrichtung
- 31: Sterilisationsgemisch
- 32: Anschlussstutzen
- 33: Anschlussflansch
- 34: Abführleitung
- 35: Sterilisationseinrichtung
- 40: Drainageeinrichtung
- 41: Drainageleitung
- 42: Eingangsöffnung
- 43: Mündungsöffnung
- 44: tiefster Abführleitungsabschnitt
- 45: Innenwandung

- 150: Heizrohrleitungswendel
- 151: Senke
- 152: Sammeleinrichtung
- 153: doppelwandige Klöpperboden
- 155: Messsonde
- 156: Drainageventil
- 157: Zwischenraum
- 158: Ableiteinrichtung
- 160: Steuereinrichtung

## Patentansprüche

1. Verdampfervorrichtung (1; 101) zum Sterilisieren von Behältern umfassend eine Verdampferkammer (4) mit einem Gehäuse (3), mit einer Zuführeinrichtung (20) zum Zuführen eines gasförmigen Mediums in die Verdampferkammer (4), mit einer Einspritzeinrichtung (8) zum Einspritzen einer flüssigen Wasserstoffperoxidlösung in die Verdampferkammer (4), mit einer Heizeinrichtung (12) zum Verdampfen der flüssigen Wasserstoffperoxidlösung innerhalb der Verdampferkammer (4), mit einer Abführeinrichtung (30) zum Abführen eines sich aus dem gasförmigen Medium und der flüssigen Wasserstoffperoxidlösung zusammensetzendes Sterilisationsgemisches (31) und mit einer Drainageeinrichtung (40) zum Ableiten zumindest von nicht verdampfter, flüssiger Wasserstoffperoxidlösung aus der Verdampferkammer (4), bei welcher die Abführeinrichtung (30) eine von der Verdampferkammer (4) wegführende Abführleitung (34) aufweist, um das Sterilisationsgemisch (31) an einer Sterilisationseinrichtung (35) bereitzustellen, und bei welcher die Drainageeinrichtung (40) eine von der Verdampferkammer (4) wegführende Drainageleitung (41) aufweist, um die nicht verdampfte, flüssige Wasserstoffperoxidlösung zur weiteren Verwendung bereitzustellen,
**dadurch gekennzeichnet, dass**
die Drainageleitung (41) der Drainageeinrichtung (40) mit ihrer Eingangsöffnung (42) an der Verdampferoberfläche(13) und somit unterseitig der Verdampferkammer (4) angeordnet ist und in die Abführleitung (34) zum Abführen des Sterilisationsgemisches (31) mündet, um zumindest die in der Verdampferkammer (4) nicht verdampfte, flüssige Wasserstoffperoxidlösung aus der Verdampferkammer (4) direkt in die Abführleitung (34) abzuführen.

2. Verdampfervorrichtung (1; 101) nach Anspruch 1,
**dadurch gekennzeichnet, dass**
die Drainageleitung (41) außerhalb der Verdampfervorrichtung (1) in die Abführleitung (34) zum Abführen des Sterilisationsgemisches (31) mündet.

3. Verdampfervorrichtung (1; 101) nach Anspruch 1 oder 2,
**dadurch gekennzeichnet, dass**
eine Mündungsöffnung (43) der Drainageleitung (41) an der Abführleitung (34) zum Abführen des Sterilisationsgemisches (31) oberhalb eines tiefsten Abführleitungsabschnittes (44) angeordnet ist.

4. Verdampfervorrichtung (1; 101) nach einem der Ansprüche 1 bis 3,
**dadurch gekennzeichnet, dass**
die Drainageeinrichtung (40) zumindest abschnittsweise die Abführleitung (34) zum Abführen des Sterilisationsgemisches (31) umfasst.

5. Verdampfervorrichtung (101) nach einem der Ansprüche 1 bis 4,
**dadurch gekennzeichnet, dass**
ein Verdampferkammeranschluss (42) der Drainageeinrichtung (40) an einer für nicht verdampfte, flüssige Wasserstoffperoxidlösung vorgesehenen Sammeleinrichtung (152) mit einer Senke (151) angeordnet ist, wobei insbesondere die Sammeleinrichtung (152) unterhalb der Verdampferkammer (4) angeordnet ist.

6. Verdampfervorrichtung (101) nach einem der Ansprüche 1 bis 5,
**gekennzeichnet durch**
eine Steuereinrichtung (160) zum Steuern der Einspritzeinrichtung (8) oder einer Dosiereinheit hiervon bezüglich einer Dosierung der flüssigen Wasserstoffperoxidlösung in die Verdampferkammer (4), wobei die Steuereinrichtung (160) ein Messelement (155) einer Detektiereinheit zum Detektieren einer Menge an nicht verdampfter, flüssiger Wasserstoffperoxidlösung an der Drainageeinrichtung (40) umfasst, wobei insbesondere das Messelement (155) in einem Abschnitt der Drainageleitung (41) zwischen der Verdampferkammer (4) und einer Mündungsöffnung (43) zwischen der Drainageleitung (41) und der Abführleitung (34) zum Abführen des Sterilisationsgemisches (31) angeordnet ist.

7. Verfahren zum Betreiben einer Verdampfervorrichtung (1; 101) zum Sterilisieren von Behältern, insbesondere einer Verdampfervorrichtung (1; 101) nach einem der vorhergehenden Ansprüche 1 bis 6, bei welchem einer Verdampferkammer (4) sowohl ein Volumengasstrom eines gasförmigen Mediums als auch eine flüssige Wasserstoffperoxidlösung zugeführt wird, bei welchem die flüssige Wasserstoffperoxidlösung innerhalb der Verdampferkammer (4) zu einem Sterilisationsgemisch (31) verdampft wird, bei welchem das Sterilisationsgemisch (31) von dem Volumengasstrom mittels einer Abführeinrichtung (30) aus der Verdampferkammer (4) abgeführt und über eine Abführleitung (34) der Abführeinrichtung (30) an einer Sterilisationseinrichtung (35) zur weiteren Verwendung bereitgestellt wird, und bei welchem nicht verdampfte, flüssige Wasserstoffperoxidlösung aus der Verdampferkammer (4) mittels einer Drainageeinrichtung (40) abgeleitet wird, um die Gefahr einer Detonation der nicht verdampften, flüssigen Wasserstoffperoxidlösung innerhalb der Verdampfervorrichtung (1; 101) zu verhindern,
**dadurch gekennzeichnet, dass**
die nicht verdampfte, flüssige Wasserstoffperoxidlösung mittels der Drainageeinrichtung (40), bei der eine Drainageleitung (41) mit ihrer Eingangsöffnung (42) an der Verdampferoberfläche(13) und somit unterseitig der Verdampferkammer (4) angeordnet ist, in, die Abführleitung (34) zum Abführen des Sterilisationsgemisches (31) eingeleitet wird.

## Claims

1. An evaporator apparatus (1; 101) for the sterilization of containers comprising an evaporator chamber (4) with a housing (3), with a supply device (20) for supplying a gaseous medium into the evaporator chamber (4), with an injection device (8) for injecting a liquid hydrogen peroxide solution into the evaporator chamber (4), with a heating device (12) for evaporating the liquid hydrogen peroxide solution inside the evaporator chamber (4), with a removal device (30) for removing a sterilization mixture (31) formed from the gaseous medium and the liquid hydrogen peroxide solution and with a drainage device (40) for the drainage at least of non-evaporated liquid hydrogen peroxide solution from the evaporator chamber (4), in which the removal device (30) has a removal line (34) leading from the evaporator chamber (4) in order to make the sterilization mixture (31) available on a sterilization device (35), and in which the drainage device (40) has a drainage line (41) leading away from the evaporator chamber (4) in order to make the non-evaporated liquid hydrogen peroxide solution available for further use, **characterized in that** the drainage line (41) of the drainage device (40) is arranged at the evaporator surface (13) by means of its inlet opening (42) and therefore at the lower side of the evaporator chamber (4) and opens into the removal line (34) for the removal of the sterilization mixture (31) in order to remove at least the liquid hydrogen peroxide solution not evaporated in the evaporator chamber (4) out of the evaporator chamber (4) directly into the removal line (34).

2. An evaporator apparatus (1; 101) according to claim 1, **characterized in that** the drainage line (41) opens outside the evaporator apparatus (1) into the removal line (34) for the removal of the sterilization mixture (34).

3. An evaporator apparatus (1; 101) according to claim 1 or 2, **characterized in that** an aperture opening (43) of the drainage line (41) is situated on the removal line (34) for the removal of the sterilization mixture (31) above a lowermost portion (44) of the removal line.

4. An evaporator apparatus (1; 101) according to any one of claims 1 to 3, **characterized in that** the drainage device (40) comprises at least in sections the removal line (34) for the removal of the sterilization mixture (31).

5. An evaporator apparatus (101) according to any one of claims 1 to 4, **characterized in that** an evaporator chamber connection (42) of the drainage device (40) is arranged on a collecting device (152) - provided for non-evaporated liquid hydrogen peroxide solution - with a drain (151), wherein the collecting device (152) in particular is arranged below the evaporator chamber (4).

6. An evaporator apparatus (101) according to any one of claims 1 to 5, **characterized by** a control device (160) for controlling the injection device (8) or a metering unit thereof with respect to a metering of the liquid hydrogen peroxide solution into the evaporator chamber (4), wherein the control device (160) comprises a measuring element (155) of a detection unit for detecting a quantity of non-evaporated liquid hydrogen peroxide solution on the drainage device (40), wherein, in particular, the measuring element (155) is arranged in a portion of the drainage line (41) between the evaporator chamber (4) and an aperture opening (43) between the drainage line (41) and the removal line (34) for the removal of the sterilization mixture (31).

7. A method of operating an evaporator apparatus (1; 101) for the sterilization of containers, in particular an evaporator apparatus (1; 101) according to any one of the preceding claims 1 to 6, in which both a volumetric gas flow of a gaseous medium and a liquid hydrogen peroxide solution are supplied to an evaporator chamber (4), in which the liquid hydrogen peroxide solution is evaporated inside the evaporator chamber (4) to form a sterilization mixture (31), in which the sterilization mixture (31) is removed from the volumetric gas flow by means of a removal device (30) out of the evaporator chamber (4) and is made available by way of a removal line (34) of the removal device (30) on a sterilization device (35) for further use, and in which non-evaporated liquid hydrogen peroxide solution is drained from the evaporator chamber (4) by means of a drainage device (40) in order to prevent the risk of detonation of the non-evaporated liquid hydrogen peroxide solution inside the evaporator chamber (1; 101), **characterized in that** the non-evaporated liquid hydrogen peroxide solution is introduced by means of the drainage device (40) into the removal line (34) for the removal of the sterilization mixture (31), a drainage line (41) being arranged at the evaporator surface (13) by means of its admission opening (42) and therefore at the lower side of the evaporator chamber (4).

## Revendications

1. Système d'évaporateur (1 ; 101) pour la stérilisation de récipients, comprenant une chambre d'évaporateur (4) avec un carter (3), avec un dispositif d'amenée (20) pour l'amenée d'un fluide gazeux dans la chambre d'évaporateur (4), avec un dispositif d'injection (8) pour l'injection d'une solution de peroxyde d'hydrogène liquide dans la chambre d'évaporateur (4), avec un dispositif de chauffage (12) pour l'évaporation de la solution de peroxyde d'hydrogène liquide à l'intérieur de la chambre d'évaporateur (4), avec un dispositif d'échappement (30) pour l'échappement d'un mélange de stérilisation (31) composé du fluide gazeux et de la solution de peroxyde d'hydrogène liquide, et avec un dispositif de drainage (40) pour l'évacuation d'au moins la solution de peroxyde d'hydrogène liquide non évaporée hors de la chambre d'évaporateur (4), où le dispositif d'échappement (30) présente une conduite d'échappement (34) partant de la chambre d'évaporateur (4) pour refouler le mélange de stérilisation (31) vers un dispositif de stérilisation (35), et où le dispositif de drainage (40) comporte une conduite de drainage (41) partant de la chambre d'évaporateur (4) pour rendre disponible la solution de peroxyde d'hydrogène liquide non évaporée pour une autre utilisation,
**caractérisé en ce que**
la conduite de drainage (41) du dispositif de drainage (40) est disposée contre la surface d'évaporateur (13) par son orifice d'admission (42), et par conséquent en dessous de la chambre d'évaporateur (4), et débouche dans la conduite d'échappement (34) pour l'échappement du mélange de stérilisation (31), afin de refouler hors de la chambre d'évaporateur (4) directement vers la conduite d'échappement (34) au moins la solution de peroxyde d'hydrogène liquide non évaporée dans la chambre d'évaporateur (4).

2. Système d'évaporateur (1 ; 101) selon la revendication 1,
**caractérisé en ce que**
la conduite de drainage (41) débouche à l'extérieur du système d'évaporateur (1) dans la conduite d'échappement (34) pour l'échappement du mélange de stérilisation (31).

3. Système d'évaporateur (1 ; 101) selon la revendication 1 ou 2,
**caractérisé en ce**
**qu'**une ouverture de sortie (43) de la conduite de drainage (41) est située contre la conduite d'échappement (34) pour l'échappement du mélange de stérilisation (31), au-dessus d'une partie inférieure (44) de la conduite d'échappement.

4. Système d'évaporateur (1 ; 101) selon l'une des revendications 1 à 3,
**caractérisé en ce que**
le dispositif de drainage (40) comprend au moins en sections la conduite d'échappement (34) pour l'échappement du mélange de stérilisation (31).

5. Système d'évaporateur (101) selon l'une des revendications 1 à 4,
**caractérisé en ce**
**qu'**un raccord (42) de chambre d'évaporateur du dispositif de drainage (40) est disposé par un puisard (151) contre un dispositif collecteur (152) prévu pour la solution de peroxyde d'hydrogène liquide non évaporée, le dispositif collecteur (152) étant notamment situé en dessous de la chambre d'évaporateur (4).

6. Système d'évaporateur (101) selon l'une des revendications 1 à 5,
**caractérisé par**
un dispositif de commande (160) pour la commande du dispositif d'injection (8) ou une unité de dosage de dosage de celui-ci pour un dosage de la solution de peroxyde d'hydrogène liquide dans la chambre d'évaporateur (4), le dispositif de commande (160) comprenant un élément de mesure (155) d'une unité de détection pour la détection d'une quantité de solution de peroxyde d'hydrogène liquide non évaporée sur le dispositif de drainage (40), l'élément de mesure (155) étant notamment disposé dans une section de la conduite de drainage (41) entre la chambre d'évaporateur (4) et une ouverture de sortie (43) de la conduite de drainage (41) sur la conduite d'échappement (34) pour l'échappement du mélange de stérilisation (31).

7. Procédé de fonctionnement d'un système d'évaporateur (1 ; 101) pour la stérilisation de récipients, en particulier d'un système d'évaporateur (1 ; 101) selon l'une des revendications 1 à 6, où un flux d'un fluide gazeux et une solution de peroxyde d'hydrogène liquide sont amenés vers une chambre d'évaporateur (4), où la solution de peroxyde d'hydrogène liquide est évaporée à l'intérieur de la chambre d'évaporateur (4) pour former un mélange de stérilisation (31), où le mélange de stérilisation (31) du flux gazeux est évacué de la chambre d'évaporateur (4) au moyen d'un dispositif d'échappement (30) et est délivré par une conduite d'échappement (34) du dispositif d'échappement (30) à un dispositif de stérilisation (35) pour une autre utilisation, et où la solution de peroxyde d'hydrogène liquide non évaporée est évacuée de la chambre d'évaporateur (4) au moyen d'un dispositif de drainage (40) pour éviter le risque de détonation de la solution de peroxyde d'hydrogène liquide non évaporée à l'intérieur du système d'évaporateur (1 ; 101),
**caractérisé en ce que**
la solution de peroxyde d'hydrogène liquide non évaporée est introduite dans la conduite d'échappement (34) pour l'échappement du mélange de stérilisation (31) au moyen du dispositif de drainage (40), où une conduite de drainage (41) est disposée contre la surface d'évaporateur (13) par son orifice d'admission (42), et par conséquent en dessous de la chambre d'évaporateur (4).
